Europäisches Patentamt

European Patent Office

Office européen des brevets

(19)

(11) Publication number: **0 070 860**
**B1**

(12) **EUROPEAN PATENT SPECIFICATION**

(45) Date of publication of patent specification: **10.04.85**

(21) Application number: **82900471.2**

(22) Date of filing: **21.12.81**

(86) International application number:
**PCT/US81/01709**

(87) International publication number:
**WO 82/02712 19.08.82 Gazette 82/20**

(51) Int. Cl.⁴: **C 07 C 67/00, C 07 C 69/14, C 07 C 69/16**

(54) **PROCESS FOR HYDROGENATING ACETIC ANHYDRIDE USING A CATALYST CONTAINING A NOBLE METAL.**

(30) Priority: **30.01.81 US 229808**
**30.01.81 US 230172**

(43) Date of publication of application:
**09.02.83 Bulletin 83/06**

(45) Publication of the grant of the patent:
**10.04.85 Bulletin 85/15**

(84) Designated Contracting States:
**BE DE FR GB NL**

(56) References cited:
**GB-A-1 538 782**
**US-A-3 579 566**
**US-A-3 957 827**
**US-A-4 221 918**

(73) Proprietor: **EASTMAN KODAK COMPANY**
**343 State Street**
**Rochester New York 14650 (US)**

(72) Inventor: **POLICHNOWSKI, Stanley Whitmore**
**547 Rambling Road**
**Kingsport, TN 37664 (US)**
Inventor: **LARKINS, Thomas Hassell, Jr.**
**Rt. 8, 4408 Beechcliff Drive**
**Kingsport, TN 37664 (US)**

(74) Representative: **Pepper, John Herbert et al**
**KODAK LIMITED Patent Department P.O. Box**
**114 190 High Holborn**
**London WC1V 7EA (GB)**

Courier Press, Leamington Spa, England.

0 070 860

**Description**

This invention relates to the hydrogenation of acetic anhydride in the presence of a catalyst containing rhodium. More specifically, this invention relates to contacting hydrogen gas with acetic anhydride in liquid phase in the presence of a homogeneous catalyst combination containing rhodium to obtain ethylidene diacetate.

Ethylidene diacetate is a valuable chemical intermediate and may be used, for example, in the preparation of vinyl acetate. In recent years, interest has increased in developing new and commercially feasible processes for preparing ethylidene diacetate. One reason for such interest is that this chemical is presently derived principally from petroleum which is relatively expensive and frequently in short supply.

It is known that ethylidene diacetate can be prepared by the hydrogenation of acetic anhydride in the presence of noble metal containing homogeneous catalysts. However, very little prior art pertaining to such processes exists and the few processes described in the art have significant disadvantages. For example, U.S. Patent 3,579,566, issued May 18, 1971 describes the hydrogenation of acetic anhydride to predominantly form ethylidene diacetate using a noble metal containing homogeneous catalyst comprising a complex of a Group VIII noble metal and a biphyllic ligand of phosphorus, arsenic or antimony. Unfortunately slow reaction rates are obtained using the catalysts disclosed in U.S. Patent 3,579,566. Furthermore, it is known that organic catalyst components such as the organic phosphines employed in the aforementioned U.S. patent are subject to degradation upon prolonged exposure to the temperatures and other reaction conditions normally employed in the hydrogenation of acetic anhydride.

This results, particularly in continuous operations, in the build-up of unwanted by-products known as "soot" or "tar" which cannot be easily separated from the catalyst present in the reaction medium. This makes it very difficult to achieve efficient catalyst recovery which is necessary as the noble metal containing catalysts are very expensive.

This invention provides a process for hydrogenating acetic anhydride which comprises contacting hydrogen gas with acetic anhydride in liquid phase in the presence of a homogeneous catalyst that is a combination of a rhodium compound with methyl iodide and lithium iodide.

It will be evident from the following description that the process of this invention provides a means for obtaining ethylidene diacetate in reasonable quantities at good to excellent space-time yields. Space-time yield is the yield of desired product in a unit of time per unit of liquid volume. It is conveniently expressed in grams (of product)/litre (of liquid volume)/hour (of reaction). In addition, methyl iodide which is employed as an organic component of the homogeneous catalysts used in the process of this invention, is much less susceptible to degradation than organic phosphine components of the type disclosed in U.S. Patent 3,579,566. This results in the formation of less "soot" or "tar" and simplifies the catalyst recovery problem previously discussed.

The rhodium catalysts employed in this invention are "homogeneous catalysts". Such catalysts are known in the art and identified as "homogeneous" because they are soluble in the liquid reaction phase. This is in contrast to noble metal containing heterogeneous catalysts which are insoluble in such phase. The homogeneous catalysts are typically formed by mixing a noble metal compound, e.g., an inorganic compound such as $RhCl_3 \cdot xH_2O$ with an iodine component or components, e.g., methyl iodide and/or lithium iodide, as illustrated by U.S. Patent 4,046,807, issued September 6, 1977, European Patent Application Publication No. 0008396, published March 5, 1980 and Japanese Published Patent Application No. 75-47922, published April 28, 1977. Examples of suitable rhodium compounds include rhodium halides such as $RhCl_3$, $RhBr_3$ and $RhI_3$, rhodium carbonyl halides such as $Rh_2(CO)_4Br_2$, $Rh_2(CO)_4Cl_2$, and $Rh_2(CO)_4I_2$ and rhodium oxides such as $Rh_2O_3$.

An effective catalyst that can be employed in the practice of this invention is a homogeneous rhodium catalyst which is formed by contacting the rhodium compound such as a rhodium halide or oxide with the iodine compounds in the presence of carbon monoxide. The carbon monoxide can conveniently be supplied in a gas stream fed to the hydrogenation zone or it can be derived as a decomposition product from the acetic anhydride present in the zone. A carbon monoxide pressure of 10 to 500 psig (170.3 to 3,548.9 kPa) can be maintained during the hydrogenation reaction to supply the carbon monoxide to the catalyst, although a carbon monoxide partial pressure can inhibit the hydrogenation reaction rate when very high total pressures are employed in practicing this invention. (The pressures indicated in kPa herein are absolute pressures rather than gauge pressures).

The concentration of the rhodium employed in the process of this invention can vary substantially depending upon such factors as the temperature, pressure and the desired space-time yield. However, when used at appropriate temperatures and pressures as indicated herein, rhodium concentrations (as rhodium metal) in the range of 50 to 5000 ppm, preferably 500 to 2500 ppm, based upon the acetic anhydride to be hydrogenated, are employed in practicing this invention. The specific rhodium compound that is introduced into the hydrogenation zone is not particularly critical so long as it is soluble in the liquid phase (the reaction medium) or provides a soluble catalyst containing rhodium, i.e. a homogeneous catalyst.

The amount of methyl iodide employed can be in the range of 5 to 35, preferably 10 to 20, weight percent, based on the weight of acetic anhydride to be hydrogenated. The absence of lithium iodide from the catalyst results in the formation of very large (and unacceptable) amounts of acetic acid. Accordingly,

2

the concentration of lithium iodide should be in the range of 0.1 to 5, preferably 0.3 to 3 weight percent, based on the weight of material to be hydrogenated. The mole ratio of lithium iodide to rhodium is normally in the range of 4 to 300 and preferably in the range of 5 to 100. The lithium iodide can be added to the hydrogenation zone as such or it can be generated in the reaction mixture from suitable lithium compounds such as lithium hydroxide, lithium carbonate or lithium acetate.

The process of this invention is carried out with the acetic anhydride in liquid phase. A solvent or diluent which is inert to the reactants can be used if desired. If a solvent or diluent is used, the conventional solvents or diluents normally used with noble metal containing homogeneous catalysts give satisfactory results. Acetic acid is an example of such a solvent or diluent and it can be added to the hydrogenation zone with the acetic anhydride. However, since acetic acid is formed as a co-product during the process it is generally not necessary to add acetic acid to the hydrogenation zone from an external source.

The temperature and pressure employed in the process of this invention are interrelated with respect to the reaction rate and also depend, to a large extent, upon the concentration of the catalyst. In general, the process is run at relatively mild, but somewhat elevated temperatures and pressures. Satisfactory results are usually obtained with temperatures in the range of 100 to 225°C, preferably 150° to 190°C and pressures (total reaction pressures) in the range of 100 to 5000 psig (790.9 to 34,576.4 kPa), preferably 500 to 3000 psig (3,548.9 to 20,786.4 kPa). The pressures can be maintained by the hydrogen gas or a mixture of hydrogen gas with another gas, e.g. carbon monoxide, that is added to the hydrogenation zone.

The hydrogenation process of this invention is performed as a batch operation or, more suitably, as a continuous operation wherein acetic anhydride is continuously fed to a hydrogenation zone and reaction mixture containing ethylidene diacetate is continuously removed. Unreacted materials and the co-product acetic acid resulting from hydrogenation can be removed from the reaction zone, separated in a distillation unit, and recycled along with any catalyst and iodides present, back to the reaction zone.

The process of the invention is further illustrated by the following example.

Example

Acetic anhydride can be effectively hydrogenated to form ethylidene diacetate in the presence of a catalytic amount of a homogeneous catalyst combination of a rhodium compound, methyl iodide and lithium iodide using varying reaction conditions and amounts of catalyst components. Also, as previously indicated herein, carbon monoxide can be added to the reaction zone with hydrogen but, as illustrated by Run 3, this is an optional feature of the invention.

To illustrate, acetic anhydride (800 g) was hydrogenated for 2 hours in the presence of a catalyst combination of a rhodium compound added as $RhCl_3 \cdot xH_2O$, with methyl iodide and lithium iodide, and acetic acid (100 g) using the temperatures and total pressures shown in the Table. The acetic anhydride, acetic acid, rhodium chloride, methyl iodide and lithium iodide were put into a 1.8 litre autoclave fitted with a stirrer. The autoclave was purged with carbon monoxide and then carbon monoxide was added to provide the pressure indicated in the Table Initial CO, psig (kPa). The autoclave was sealed, heated and the contents stirred until the reaction temperature was reached, at which time hydrogen gas was added to increase the autoclave internal pressure to a predetermined value. The time at which the autoclave internal pressure reached the predetermined value was taken as the start of the 2 hour reaction time. The pressure in the autoclave was maintained at the preset value during each run by adding hydrogen gas at the same rate at which it was consumed. When the reaction time elapsed the autoclave was cooled using a stream of cold air. Gas was vented from the autoclave and the reaction product was analyzed by gas chromatographic methods.

The Table shows the temperature (°C) and pressure (Initial CO and Total, psig (kPa)) used, the amounts of $RhCl_3 \cdot xH_2O$ (g) and methyl and lithium iodides ($CH_3I$, LiI, g) added to the autoclave in each run, the amounts (in moles) of acetic acid (HOAc) and ethylidene diacetate (EDA) produced, the amount of acetic anhydride ($Ac_2O$, in moles) recovered, and the percent yield of ethylidene diacetate (EDA). The percent yield EDA was determined by measuring, by the gas chromatographic method, the amount of $Ac_2O$ present in the autoclave at the start of the reaction when the reaction temperature was reached. The amount of $Ac_2O$ was again measured at the end of the two hour reaction period (amount $Ac_2O$ recovered). The difference in these two measured values was divided by two in order to account for the stoichiometry of the reaction and determine the value for a 100% theoretical EDA yield under the conditions of reaction. The value for the amount of EDA recovered was then divided by the value of the 100% theoretical EDA yield and converted to percent.

3

TABLE

| Run | Temp., °C | Initial CO pressure, psig (kPa) | Total pressure psig (kPa) | $RhCl_3 \cdot xH_2O$, g | LiI, g | $CH_3I$, g | EDA moles | HoAc, moles | $Ac_2O$, moles | % Yield EDA |
|-----|-----------|----------------------------------|----------------------------|--------------------------|--------|-------------|-----------|-------------|-----------------|-------------|
| 1 | 175 | 100 (790.9) | 1500 (10,443.8) | 0.62 | 3.18 | 100 | 0.72 | 0.84 | 0.78 | 92 |
| 2 | 175 | 100 (790.9) | 2500 (17,338.9) | 0.62 | 3.18 | 100 | 1.12 | 1.22 | 1.32 | 85 |
| 3 | 175 | 0 | 1500 (10,443.8) | 0.62 | 3.18 | 100 | 0.76 | 0.93 | 0.98 | 77 |
| 4 | 175 | 100 (790.9) | 1500 (10,443.8) | 0.62 | 3.18 | 50 | 0.41 | 0.45 | 0.41 | 100 |
| 5 | 130 | 100 (790.9) | 2500 (17,338.9) | 2.48 | 12.70 | 100 | 0.81 | 0.81 | 0.84 | 97 |

# 0 070 860

## Claims

1. A process for hydrogenating acetic anhydride which comprises contacting hydrogen gas with acetic anhydride in liquid phase in the presence of a homogeneous catalyst that is a combination of a rhodium compound with methyl iodide and lithium iodide.

2. The process according to claim 1 wherein the amount of methyl iodide is 5 to 35 weight percent, and the amount of lithium iodide is 0.1 to 5 weight percent, based on the weight of acetic anhydride.

3. The process according to claim 2 wherein the hydrogen gas is contacted with acetic anhydride at a temperature of 100° to 225°C and a pressure of 790.9 to 34,576.4 kPa.

4. The process according to claim 1 wherein the acetic anhydride is contacted with a mixture of hydrogen and carbon monoxide gases.

5. The process according to claim 3 wherein the hydrogen is contacted with acetic anhydride at a temperature of 150° to 190°C and a pressure of 3,548.9 to 20,786.4 kPa, the amount of methyl iodide is 10 to 20 weight percent, and the amount of lithium iodide is 0.3 to 3 weight percent, based on the weight of acetic anhydride.

## Patentansprüche

1. Verfahren zur Hydrierung von Essigsäureanhydrid, bei dem man Wasserstoffgas mit Essigsäureanhydrid in flüssiger Phase in Gegenwart eines homogenen Katalysators aus einer Kombination aus einer Rhodiumverbindung mit Methyliodid und Lithiumiodid in Kontakt bringt.

2. Verfahren nach Anspruch 1, dadurch gekennzeichnet, daß die Menge an Methyliodid bei 5 bis 35 Gew.-% und die Menge an Lithiumiodid bei 0,1 bis 5 Gew.-%, bezogen auf das Gewicht an Essigsäureanhydrid, liegt.

3. Verfahren nach Anspruch 2, dadurch gekennzeichnet, daß das Wasserstoffgas mit Essigsäureanhydrid bei einer Temperatur von 100 bis 225°C und einem Druck von 790,9 bis 34576,4 kPa in Kontakt gebracht wird.

4. Verfahren nach Anspruch 1, dadurch gekennzeichnet, daß das Essigsäureanhydrid mit einer Mischung aus Wasserstoff und Kohlenmonoxidgasen in Kontakt gebracht wird.

5. Verfahren nach Anspruch 3, dadurch gekennzeichnet, daß der Wasserstoff mit Essigsäureanhydrid bei einer Temperatur von 150 bis 190°C und einem Druck von 3548,9 bis 20786,4 kPa in Kontakt gebracht wird, und daß die Menge an Methyliodid bei 10 bis 20 Gew.-% und die Menge an Lithiumiodid bei 0,3 bis 3 Gew.-%, bezogen auf das Gewicht des Essigsäureanhydrids, liegt.

## Revendications

1. Procédé pour hydrogéner l'anhydride acétique qui consiste à mettre en contact l'hydrogène gazeux avec l'anhydride acétique en phase liquide en présence d'un catalyseur homogène qui est une combinaison d'un composé du rhodium avec l'iodure de méthyle et l'iodure de lithium.

2. Procédé conforme à la revendication 1, dans lequel la quantité d'iodure de méthyle est de 5 à 35% en masse, et la quantité d'iodure de lithium est de 0,1 à 5% en masse, par rapport à la masse d'anhydride acétique.

3. Procédé conforme à la revendication 2, dans lequel on met en contact l'hydrogène gazeux avec l'anhydride acétique à une température de 100° à 225°C et à une pression de 790,9 à 34 576,4 kPa.

4. Procédé conforme à la revendication 1, dans lequel on met en contact l'anhydride acétique avec un mélange d'hydrogène et de monoxyde de carbone gazeux.

5. Procédé conforme à la revendication 3, dans lequel on met en contact l'hydrogène avec l'anhydride acétique à une température de 150° à 190°C et à une pression de 3548,9 à 20 786,4 kPa, la quantité d'iodure de méthyle est de 10 à 20% en masse, et la quantité d'iodure de lithium est de 0,3 à 3% en masse, par rapport à la masse d'anhydride acétique.